# EUROPEAN PATENT SPECIFICATION

(11) **EP 0 728 443 B1**
(45) Date of publication and mention of the grant of the patent: **09.08.2000**
(21) Application number: 96103815.5
(22) Date of filing: 09.05.1989
(51) Int. Cl.: A61B 8/00, G01N 29/00, A61B 8/08

(54) **Ultrasonic densitometer and method**
Ultraschall-Dichtemessgerät und Verfahren
Appareil de mesure de la densité à ultrason et procédé

(30) Priority: 11.05.1988 US 193295
(43) Date of publication of application: 28.08.1996
(62) Divisional of application: 89304664.9
(73) Proprietor: LUNAR CORPORATION, Madison Wisconsin 53713 (US)
(72) Inventor: Rossman, Phillip J., Rochester, Minnesota 55901 (US); Weiner, Scott A., Mt. Horeb, Wisconsin 53572 (US)
(74) Representative: Ford, Michael Frederick

(56) References cited:
- WO-A-87/07494
- US-A- 4 457 311
- ENGINEERING IN MEDICINE, vol. 13, no. 2, 1984, LONDON, GB, pages 89-91, XP000569604 C.M.LANGTON ET AL: "The measurement of broadband ultrasonic attenuation in cancellous bone"
- IEEE TRANSACTIONS ON BIOMEDICAL ENGINEERING, vol. bme-31, no. 12, December 1984, NEW YORK, US, pages 878-884, XP000195701 J.L.KATZ ET AL: "The Structure and Anisotropic Mechanical Properties of Bone"

## Description

### Background of the Invention

### 1. Field of the Invention

The present invention relates to devices which are used for measuring the density of the os calcis, in particular to devices which utilize ultrasonic acoustic signals to measure the physical properties and integrity of the os calcis.

### 2. Description of the Prior Art

Various devices presently exist which may be used to measure the physical properties and integrity of a member such as a bone. Non-invasive density measuring devices can be used to determine cumulative internal damage caused by micro-crushing and micro- fracturing occurring in the bones of humans or animals such as race horses. Additionally, osteoporosis, or loss of bone mineralization, detection in humans and its cure or prevention are increasingly becoming areas of intense medical and biological interest. As the average age of the human population increases, a greater number of patients are developing complications due to rapid trabecular bone loss.

U.S. Patent No. 3,847,141 to Hoop discloses a device for measuring the density of a bone structure, such as a finger bone or heel bone, to monitor the calcium content thereof. The device includes a pair of opposed spaced ultrasonic transducers which are held within a clamping device clamped on the bone being analyzed. A pulse generator is coupled to one of the transducers to generate an ultrasonic soundwave which is directed through the bone to the other transducer. An electric circuit couples the signals from the receive transducer back to the pulse generator for retriggering the pulse generator in response to those signals. The pulses therefore are produced at a frequency proportional to the transit time that the ultrasonic wave takes to travel through the bone structure, which is directly proportional to the speed of the sound through the bone. The speed of sound through a bone has been found to be proportional to the density of the bone. Thus the frequency at which the pulse generator is retriggered is proportional to the density of the bone.

Another device and method for establishing, in vivo the strength of a bone is disclosed in U.S. Patents Nos. 4,361,154 and 4,421,119 to Pratt, Jr. The device includes a launching transducer and a receiving transducer which are connected by a graduated vernier and which determine the speed of sound through the bone to determine its strength. The vernier is used to measure the total transit distance between the surfaces of the two transducers.

U.S. Patent No. 4,457,311 discloses an apparatus for ultrasound scanning of the human back, in which there are a linear array of transducers and a transporter system for moving the transducers along the back. In operation, each transducer sequentially emits a pulse and is then immediately switched to receiving mode. The emitted pulse is reflected and the reflected pulse is detected by the same transducer. This is repeated along the length of the array, building up a series of reflective measurements which are processed and from which an output representative of the body structure scanned can be produced.

Lees (Lees, S. (1986) Sonic Properties of Mineralized Tissue, Tissue Characterization With Ultrasound, CRC publication 2, pp. 207-226) discusses various studies involving attenuation and speed of sound measurements in both cortical and spongy (cancellous or trabecular) bone. The results of these studies reveal a linear relationship between the wet sonic velocity and wet cortical density, and between the dry sonic velocity and the dry cortical density. The transit times of an acoustic signal through a bone member therefore are proportional to the bone density. Langton et al (Langton, C M, Palmer, S D and Porter S W (1984) The Measurement of Broad Band Ultrasonic Attenuation in Cancellous Bone, Eng Med, 13, 89-91) published the results of a study of ultrasonic attenuation versus frequency in the os calcis (heel bone) that utilised through transmission techniques. These authors suggested that attenuation differences observed in different subjects were due to changes in the mineral content of the os calcis. They also suggested that low frequency ultrasonic attenuation may be parameter useful in the diagnosis of osteoporosis or as a predictor of possible fracture risk.

WO 87/07494 discloses apparatus and a method for assessing bone condition in which a test is carried out using a single pair of transducers for sending and receiving ultrasound pulses and measuring the transit time through bone. The measurements are compared with a database of velocity values relating to different patients which can be categorised by a number of factors e.g. age, sex, race, known bone conditions in order to identify such a known patient with similar values in order to make a diagnosis. However, as the apparatus of WO 87/07494 has only one transducer for sending a pulse and one transducer for receiving a pulse, the positioning of the transducers is critical when comparing repeated measurements from a single patient or measurements between patients.

A primary object of the invention is to provide an ultrasound densitometer device and method for measuring the physical properties and integrity of the os calcis in vivo.

An object of the invention is to provide an ultrasound densitometer device and method for measuring the physical properties and integrity of the os calcis in vivo by determining the absolute attenuation of specific frequency components of ultrasound acoustic signals through the heel.

A further object of the invention is to provide an ultrasound densitometer device and method for measuring the physical properties and integrity of the os calcis in vivo by comparing the absolute attenuation of specific frequency components of ultrasound acoustic signals through the heel with the absolute attenuation of corresponding frequency components of acoustic signals through a material of known acoustic properties.

Another object of the invention is to provide an ultrasound densitometer device and method of measuring the physical properties and integrity of the os calcis in vivo by comparing the transit time of ultrasonic acoustic signals through the heel with a selected normal transmit time selected from a database of normal transit times which are dependent upon the age, height, weight, race, or sex of an individual being tested, and also upon the distance between the transducers or the size of the heel.

According to a first aspect of the invention, there is provided an ultrasonic densitometer for investigating the os calcis in vivo comprising:
a transmission ultrasonic transducer array and a reception ultrasonic transducer array spaced from the transmission array so as to receive a heel there between;
electrical means connected to the transmission and reception transducer arrays for launching an ultrasonic pulse from the transmission array, sensing the receipt of the pulse to the reception array and measuring the transit time of the pulse passing through the heel when the heel is positioned in the path of the ultrasonic pulse;
an electronic means including a microprocessor operating according to a stored program to:
   (i) select one of a plurality of normal transit times from an electronic database of normal transit times;
   (ii) make a mathematical comparison of said member transit time of the pulse passing through the heel to the selected normal transit time; and
   (iii) relate said mathematical comparison to the physical properties and integrity of the os calcis of said heel.

According to a second aspect of the invention there is provided a method for measuring the physical properties and integrity of the os calcis in vivo, comprising the steps of:
(a) transmitting an acoustic signal through the heel using a transmission ultrasonic transducer array and a reception ultrasonic transducer array spaced from the transmission array, the transmission array launching an ultrasonic pulse and the reception array receiving the pulse after passage through the heel;
(b) determining a heel transit time of the acoustic signal through the heel;
(c) selecting one of a plurality of normal transit times from a database of normal transit times;
(d) making a mathematical comparison of said heel transit time to the selected normal transit time; and
(e) relating said mathematical comparison to the physical properties and integrity of the os calcis.

Other objects, features and advantages of the invention will be apparent from the following detailed description taken in conjunction with the accompanying drawings, which exemplify a preferred embodiment of the invention.

### Brief Description of the Drawings

Fig. 1 is a perspective view of the ultrasound densitometer device constructed in accordance with the present invention.

Fig. 2 is a perspective view of an acoustic coupler, two of which are shown in Fig. 1.

Fig. 3 is a front view of a transducer face from which acoustic signals are transmitted or by which acoustic signals are received, the face of the other transducer being the mirror image thereof.

Fig. 4 is a schematic block diagram view of the circuitry of the ultrasound densitometer device constructed in accordance with the present invention.

Fig. 5 illustrates the method of sampling a received waveform used by the circuit of Fig. 4.

Fig. 6 is a sample of an actual ultrasonic pulse and response from an ultrasonic densitometer according to the present invention.

Fig. 7 is a sample plot of relative ultrasound pulse intensity over frequency range.

Fig. 8 is a graph in frequency domain illustrating the shift in alternation versus frequency characteristic of a measured object as compared to reference.

### Description of the Preferred Embodiment

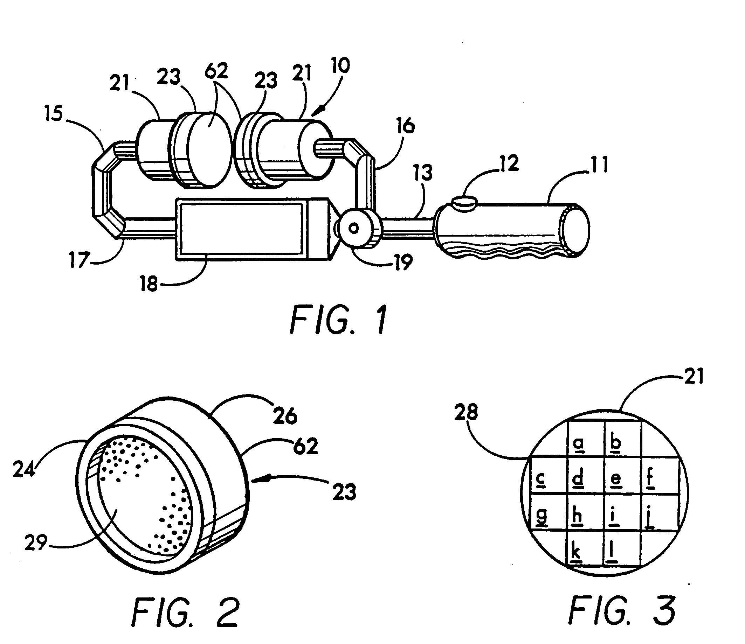

Referring more particularly to the drawings, wherein like numbers refer to like parts, Fig. 1 shows a portable ultrasound densitometer 10 for measuring the physical properties and integrity of the os calcis in vivo. The densitometer 10 as shown in Fig. 1 includes a handle 11 with actuator button 12. Extending linearly from the handle 11 is a connection rod 13. The densitometer 10 also includes a fixed arm 15 and an adjustable arm 16. The fixed arm 15 preferably is formed continuously with the connection rod 13, and therefore is connected to an end 17 of the connection rod 13. The adjustable arm 16 is slidably mounted on the connection rod 13 between the handle 11 and a digital display 18 mounted on the rod 13. The knob 19 may be turned so as to be locked or unlocked to allow the adjustable arm 16 to be slid along the connection rod 13 so that the distance between the arms 15 and 16 may be adjusted.

Connected at the end of the fixed arm 15 is a first (left) transducer 21 and at the end of the adjustable arm 16 is a second (right) transducer 21. As shown in Figs. 1 and 2, each of the transducers 21 has mounted on it a respective compliant acoustic coupler 23 to acoustically couple the transducer to the object being tested. The acoustic coupler 23 includes a plastic ring 24 and attached pad 26 formed of urethane or other compliant material. Figure 3 shows a face 28 of the first (left) transducer 21 which is normally hidden behind the compliant pad 26 of the acoustic coupler 23. The transducer face 28 normally abuts against the inner surface 29 of the pad 26 shown in Fig. 2. The transducer face 28 shown in Fig. 3 includes an array of twelve transducer elements labeled a-l. The second (right) transducer 21 includes a face 28 which is the mirror image of that shown in Fig. 3.

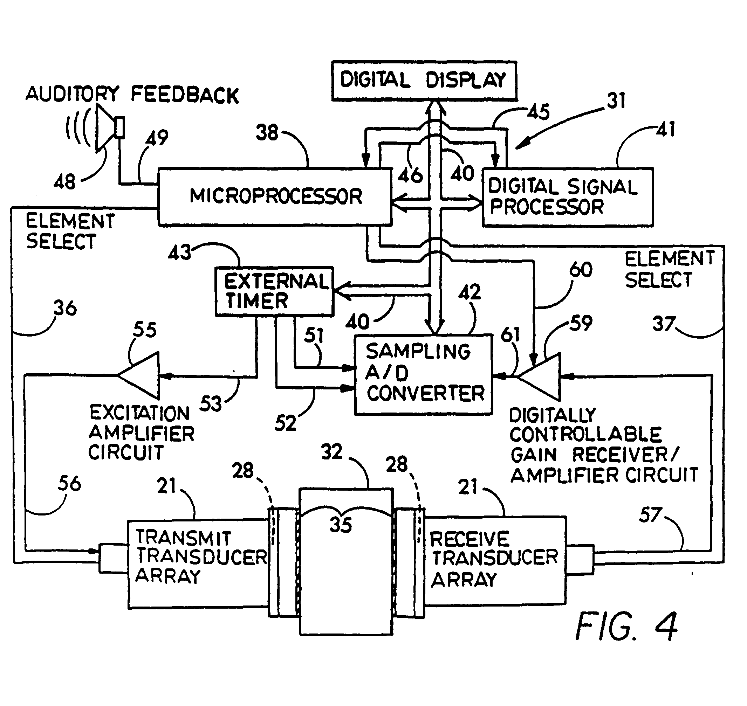

Figure 4 generally shows in schematic fashion the electronic circuitry 31 of the densitometer 10, which is physically contained in the housing of the digital display 18. An object 32 is placed between the two transducers 21 so that acoustic signals may be transmitted through the object. This object 32 represents the os calcis, or some material with known acoustic properties such as distilled water or a neoprene reference block. As shown in the embodiment illustrated in Fig. 4, the leftmost transducer 21 is a transmit transducer and the rightmost transducer 21 a receive transducer. In fact though, the arrangement of the transmit and receive transducers may be reversed. The transmit and receive transducers 21 of the circuit of Fig. 4 are connected by element select signals 36 and 37 to a microprocessor 38. The microprocessor 38 is programmed to determine which one of the respective pairs of transducer elements a through 1 are to be transmitting and receiving at any one time. This selection is accomplished by the element select signal lines 36 and 37, which may be either multiple signal lines or a serial data line to transmit the needed selection data to the transducers 21. The microprocessor 38 is also connected by a data and address bus 40 to the digital display 18, a digital signal processor 41, a sampling analog to digital converter 42, and a set of external timers 43. The microprocessor 38 has "on board" electrically programmable non-volatile random access memory (NVRAM) and, perhaps as well, conventional RAM memory, and controls the operations of the densitometer 10. The digital signal processor 41 has "on board" read-only memory (ROM) and performs many of the mathematical functions carried out by the densitometer 10 under the control of the microprocessor 38. The digital signal processor 41 specifically includes the capability to perform discrete Fourier transforms, as is commercially available in integrated circuit form presently, so as to be able to convert received waveform signals from the time domain to the frequency domain. The microprocessor 38 and digital signal processor 41 are interconnected also by the control signals 45 and 46 so that the microprocessor 38 can maintain control over the operations of the digital signal processor 41 and receive status information back. Together the microprocessor 38 and the digital signal processor 41 control the electrical circuit 31 so that the densitometer 10 can carry out its operations, which will be discussed below. An auditory feedback mechanism 48, such as an audio speaker, can be connected to the microprocessor 38 through an output signal 49.

The external timer 43 provides a series of clock signals 51 and 52 to the A/D converter 42 to provide time information to the A/D converter 42 so that it will sample at timed intervals electrical signals which it receives ultimately from the transmit transducer, in accordance with the program in the microprocessor 38 and the digital signal processor 41. The external timer 43 also create a clock signal 53 to an excitation amplifier 55. Timed pulses are generated by the timer 43 and sent through the signal line 53 to the amplifier 55 to be amplified and directed to the transmit transducer 21 through the signal line 56. The transmit transducer 21 converts the amplified pulse into an acoustic signal which is transmitted through the heel or material 32 to be received by the receive transducer 21 which converts the acoustic signal back to an electrical signal. The electrical signal is directed through output signal 57 to a receiver amplifier 59 which amplifies the electrical signal.

The excitation amplifier circuit 55 is preferably a digitally controllable circuit designed to create a pulsed output. The amplification of the pulse can be digitally controlled in steps from one to ninety-nine. In this way, the pulse can be repetitively increased in amplitude under digital control until a received pulse of appropriate amplitude is received at the receiver/amplifier circuit 59, where the gain is also digitally adjustable.

Connected to the receiver amplifier circuit 59 and integral therewith is a digitally controllable automatic gain control circuit which optimizes the sensitivity of the receive transducer 21 and the amplifer circuit 59 to received acoustic signals. The microprocessor 38 is connected-to the amplifier circuit and automatic gain control 59 through signal line 60 to regulate the amplification of the amplifier circuit and gain control 59. The amplified electric signals are directed through lead 61 to the A/D converter 42 which samples those signals at timed intervals. The A/D converter 42 therefore in effect samples the received acoustic signals. As a series of substantially identical acoustic signals are received by the receive transducer 21, the A/D converter 42 progressively samples an incremental portion of each successive signal waveform. The microprocessor 38 is programmed so that those portions are combined to form a digital composite waveform which is nearly identical to a single waveform. This digitalized waveform may be displayed on the digital display 18, or processed for numerical analysis by the digital signal processor 41.

The densitometer constructed in accordance with Figs. 1-4 is operated according to the method of the present invention, as defined in claim 6. In any event, for the method of use of the densitometer to measure the physical properties of the os calcis to be fully understood, it is first necessary to understand the internal operation of the densitometer itself.

In this method of use, the densitometer is intended to be placed on the heel whose properties are being measured. This is done by placing the transducers 21 on the opposite sides of the heel. To accomplish this, the knob 19 is loosened to allow the adjustable arm 16 to be moved so that the transducers 21 can be placed on opposite sides of the heel of a human patient. The outside surfaces of the pads 26 can be placed against the heel of the subject with an ultrasound gel 35 or other coupling material placed between the pads 26 and subject 32 to allow for improved transmission of the acoustic signals between the heel 32 and transducers 21. Once the transducers 21 are properly placed on the heel, the knob 19 may be tightened to hold the adjustable arm 16 in place, with the transducers 21 in spaced relation to each other with the heel 32 therebetween. The actuator button 12 may then be pressed so that acoustic signals will be transmitted through the heel 32 to be received by the receive transducer 21. The electronic circuit of Fig. 4 receives the electrical signals from the receive transducer 21, and samples and processes these signals to obtain information on the physical properties and integrity of the os calcis in vivo. The microprocessor 38 is programmed to indicate on the digital display 18 when this information gathering process is complete. Alternatively, the information may be displayed on the digital display 18 when the information gathering process is completed. For example, the transit time of the acoustic signals through the heel 32 could simply be displayed on the digital display 18.

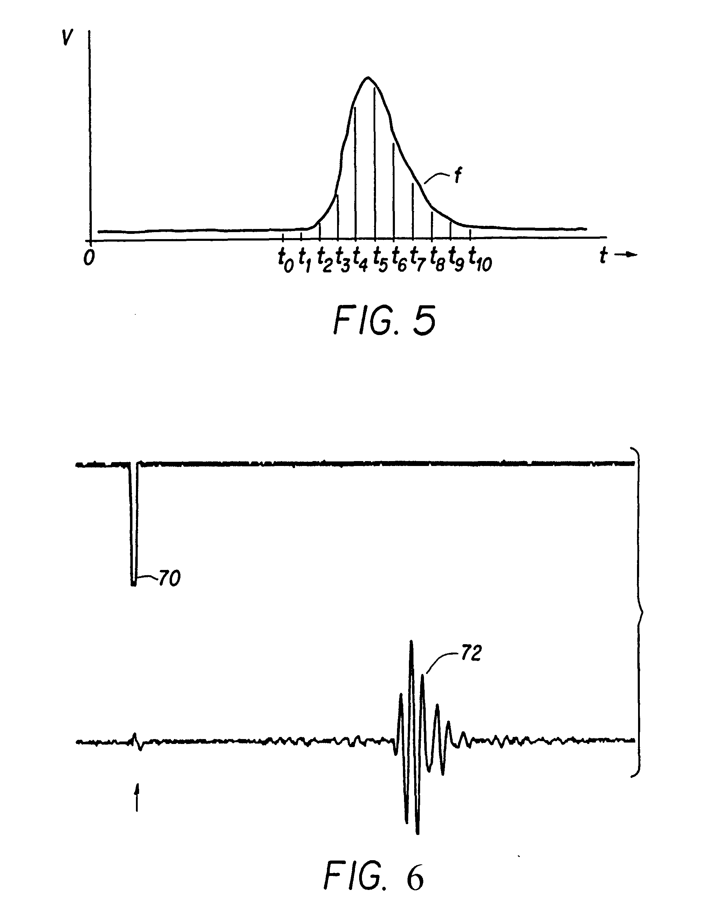

Considering in detail the operation of the circuitry of Fig. 4, the general concept is that the circuitry is designed to create an ultrasonic pulse which travels from transmit transducer 21 through the heel 32 of the subject and is then received by the receive transducer 21. The circuitry is designed to both determine the transit time of the pulse through the heel 32, to ascertain the attenuation of the pulse through the heel 32, and to be able to reconstruct a digital representation of the waveform of the pulse after it has passed through the heel 32, so that it may be analyzed to determine the attenuation at selected frequencies. To accomplish all of these objectives, the circuitry of Fig. 4 operates under the control of the microprocessor 38. The microprocessor 38 selectively selects, through the element select signal lines 36, a corresponding pair or a group of the elements a through 1 on the face of each of the transducers 21. The corresponding elements on each transducer are selected simultaneously while the remaining elements on the face of each transducer are inactive. With a given element, say for example element a selected, the microprocessor then causes the external timer 43 to emit a pulse on signal line 53 to the excitation amplifier circuit 55. The output of the excitation amplifier 55 travels along signal line 56 to element a of the transmit transducer 21, which thereupon emits the ultrasonic pulse. The corresponding element a on the receive transducer 21 receives the pulse and presents its output on the signal line 57 to the amplifier circuit 59. What is desired as an output of the A/D converter 42 is a digital representation of the analog waveform which is the output of the single transducer element which has been selected. Unfortunately, "real time" sampling A/D converters which can operate rapidly enough to sample a waveform at ultrasonic frequencies are relatively expensive. Therefore it is preferred that the A/D converter 42 be an "equivalent time" sampling A/D converter. By "equivalent time" sampling, it is meant that the A/D converter 42 samples the output of the transducer during a narrow time period after any given ultrasonic pulse. The general concept is illustrated in Fig. 5. The typical waveform of a single pulse received by the receive transducer 21 and imposed on the signal line 57 is indicated by a function "f". The same pulse is repetitively received as an excitation pulse is repetitively launched. The received pulse is sampled at a sequence of time periods labeled t₀-t₁₀. In other words, rather than trying to do a real-time analog to digital conversion of the signal f, the signal is sampled during individual fixed time periods t₀-t₁₀ after the transmit pulse is imposed, the analog value during each time period is converted to a digital function, and that data is stored. Thus the total analog wave form response can be recreated from the individual digital values created during each time period t, with the overall fidelity of the recreation of the wave form dependent on the number of time periods t which are sampled. The sampling is not accomplished during a single real time pulse from the receive transducer 21. Instead, a series of pulses are emitted from the transmit transducer 21. The external timer is constructed to provide signals to the sampling A/D converter 42 along signal lines 51 and 52 such that the analog value sampled at time period t₀ when the first pulse is applied to a given transducer element, then at time t₁ during the second pulse, time t₂ during the third pulse, etc. until all the time periods are sampled. Only after the complete waveform has been sampled for each element is the next element, i.e. element b, selected. The output from the A/D converter 42 is provided both to the microprocessor 38 and to the signal processor 41. Thus the digital output values representing the complex waveform f of Fig. 5 can be processed by the signal processor 41 after they are compiled for each transducer element. The waveform can then be analyzed for time delay or attenuation for any given frequency component with respect to the characteristic of the transmitted ultrasonic pulse. The process is then repeated for the other elements until all elements have been utilized to transmit a series of pulses sufficient to create digital data representing the waveform which was received at the receive transducer array 21. It is this data which may then be utilized in a variety of methods for determining the physical properties of the os calcis, and determining on the manner in which the densitometer is being utilized, and the data being sought, the appropriate output can be provided from either the microprocessor 38 or the signal processor 41 through the digital display 18.

Because the ultrasonic pulsing and sampling can be performed so rapidly, at least in human terms, the process of creating a sampled ultrasonic received pulse can optionally be repeated several times to reduce noise by , signal averaging. If this option is to be implemented, the process of repetitively launching ultrasonic pulses and sampling the received waveform as illustrated in Fig. 5 is repeated one or more times for each element in the array before proceeding to the next element. Then the sampled waveforms thus produced can be digitally averaged to produce a composite waveform that will have a lesser random noise component than any single sampled waveform. The number of repetitions necessary to sufficiently reduce noise can be determined by testing in a fashion known to one skilled in the art.

Having thus reviewed the internal operation of the densitometer of Figs. 1-4, it is now possible to understand the method of use of the densitometer to measure the physical properties of the os calcis. A first method of use which does not pertain to the invention involves measuring transit time of an ultrasonic pulse through the heel of a subject and comparing that time to the time an ultrasonic pulse requires to travel an equal distance in a substance of known acoustic properties such as water. For this procedure, the adjustable arm 16 is adjusted until the heel 32 of the subject is clamped between the transducers 21. Then the knob 19 is tightened to fix the adjustable arm in place. The actuator button 12 is then pressed to initiate a pulse and measurement. Next the densitometer is removed from the heel while keeping the knob 19 tight so that the distance between the transducers 21 remains the same. The device 10 is then placed about or immersed in a standard material with known acoustic properties, such as by immersion in a bath of distilled water. The actuator button 12 is pressed again so that acoustic signals are transmitted from the transmit transducer 21 through the standard material to the receive transducer 21. While it is advantageous to utilize the whole array of elements a through 1 for the measurement of the heel , it may only be necessary to use a single pair of elements for the measurement through the standard assuming only that the standard is homogeneous, unlike the heel. The signal profiles received by the two measurements are then analyzed by the microprocessor 38 and the signal processor 41. This analysis can be directed both to the comparative time of transit of the pulse through the subject as compared to the standard and to the characteristics of the waveform in frequency response and attenuation through the heel as compared to the standard.

Thus in this method which is not part of the invention the densitometer may determine the physical properties and integrity of the os calcis by both or either of two forms of analysis. The densitometer may compare the transit time of the acoustic signals through the heel with the transit time of the acoustic signals through the material of known acoustic properties, and the device 10 may also compare the attenuation as a function of frequency of the broadband acoustic signals through the heel 32 with the absolute attenuation of corresponding specific frequency components of the acoustic signals through the material of known acoustic properties. The "attenuation" of an acoustic signal through a substance is the dimunition of the ultrasonic waveform from the propogation through either the subject or the standard. The theory and experiments using both of these methods are presented and discussed in Rossman, P.J., Measurements of Ultrasonic Velocity and Attenuation In The Human Os Calcis and Their Relationships to Photon Absorptiometry Bone Mineral Measurements (1987) (a thesis submitted in partial fulfillment of the requirements for the degree of Master of Science at the University of Wisconsin-Madison). Tests have indicated that there exists a linear relationship between ultrasonic attenuation (measured in decibels) (db)) at specific frequencies, and those frequencies. The slope (dB/MHz) of the linear relationship, referred to as the broadband ultrasonic attenuation, is dependent upon the physical properties and integrity of the substance being tested. With a bone, the slope of the linear relationship would be dependent upon the bone mineral density. Thus broadband ultrasonic attenuation through a bone is a parameter directly related to the quality of the cancellous bone matrix.

For this method which is not part of the invention, the microprocessor 38 may therefore be programmed so that the device determines the physical properties and integrity of the os calcis by comparing either relative transit times and/or relative broadband ultrasonic attenuation through the heel and a material of known acoustic properties. When comparing the transit times, the microprocessor 38 may be programmed most simply so that the electronics, having received the acoustic signals after they have been transmitted through the heel, determines the heel transit time of those acoustic signals through the heel, and after the acoustic signals have been transmitted through the material of known acoustic properties, determines the "material" transit time of the acoustic signals through the material. These time periods may be measured most simply by counting the number of clock pulses of known frequency emitted by the timer 43 between the time of launching the pulse and the sensing of the received pulse at the A/D converter 42. The microprocessor 38 then makes a mathematical "time" comparison of the heel transit time to the material transit time and then relates that mathematical time comparison to the physical properties and integrity of the os calcis. The mathematical time comparison may be made by either determining a difference between the heel transit time and the material transit time, or by determining a ratio between the heel transit time and the material transit time.

As a second method of using the densitometer which is not part of the invention, it may also determine the physical properties and integrity of the os calcis 32 by determining and comparing the absolute attenuation of the broadband frequency components of the acoustic signals through the heel without reference to a material having known acoustic properties. Using this method, the comparison of velocity to a standard is not necessary and absolute transit time of the pulse need not be calculated since it is attenuation that is measured. In such a mode, it is preferable that the transmit transducer 21 transmits an acoustic signal which has a broad range of frequency components, such as a simple ultrasonic pulse. In any case, the acoustic signal should have at least one specific frequency component.

In this attenuation comparison mode, the microprocessor 38 is programmed so that after the receive transducer 21 receives the acoustic signals transmitted through the heel bone 32, it determines the absolute attenuation through the heel 32 of the frequency component spectrum of the acoustic signals. It is to facilitate the measurement of attenuation that the excitation amplifier circuit 55 and the receiver amplifier 59 have amplification levels which may be digitally controlled. By successively varying the gain of the amplifiers 55 and 59 on successive pulses, the circuit of Fig. 4 can determine what level of gain is necessary to place the peak of the received waveform at a proper voltage level. This gain is, of course, a function of the level of attenuation of the acoustic pulse during transit through the heel 32. After the receive transducer 21 receives acoustic signals, microprocessor 38 in conjunction with the signal processor 41 determines the absolute attenuation of individual specific frequency components of the received acoustic signal transmitted through the material. The digital signal processor 41 then makes mathematical "attenuation" comparisons of the corresponding individual specific frequency components through the member. A set of mathematical attenuation comparisons between corresponding frequency components may be thereby obtained, one comparison for each frequency component compared. The manner in which the attenuation functions with respect to frequency can thus be derived. The microprocessor 38 and digital signal processor 41 then relate that function to the physical properties and integrity of the os calcis.

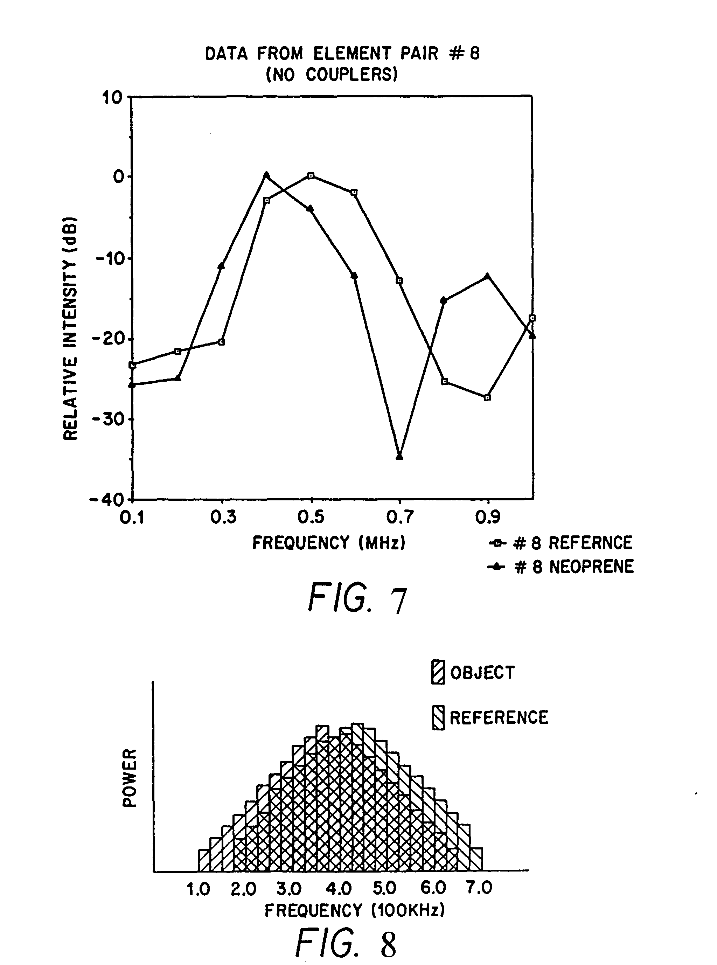

Shown in Fig. 6 is a sample broadband ultrasonic pulse and a typical received waveform. To achieve an ultrasonic signal that is very broad in the frequency domain, i.e., a broadband transmitted signal, an electronic pulse such as indicated at 70 is applied to the selected ultrasonic transducer in the transmit array 21 which then resonates with a broadband ultrasonic emission. The received signal, such as indicated at 72 in Fig. 6 in a time domain signal plot, is then processed by discrete Fourier transform analysis so that it is converted to the frequency domain. Shown in Fig. 7 is a pair of plots of sample received signals, in frequency domain plots, showing the shift in received signal intensity as a function of frequency between a reference object and a plug of neoprene placed in the instrument. Fig. 8 illustrates a similar comparison, with Fig. 7 using relative attenuation in the vertical dimension and Fig. 8 using absolute power of the received signal. Both representations illustrate the difference in relative intensities as a function of frequency illustrating how broadband ultrasonic attenuation varies from object to object. The actual value calculated, broadband ultrasonic attenuation, is calculated by first comparing the received signal against the reference signal, then performing the discrete Fourier transform to convert to frequency domain, then performing a linear regression of the difference in attenuation slope to derive broadband ultrasonic attenuation.

The mathematics of the discrete Fourier transform are such that another parameter related to bone member density may be calculated in addition to, or in substitution for, attenuation. When the discrete Fourier transform is performed of the time-domain signal, the solution for each point includes a real member component and an imaginary member component. The values graphed in Figs. 7 and 8 are the amplitude of the received pulse as determined from this discrete Fourier transform by taking the square root of the sum of the squares of the real component and the imaginary component. The phase angle of the change in phase of the ultrasonic pulse as it passed through the member can be calculated by taking the arctangent of the ratio of the imaginary to the real components. This phase angle value is also calculated to bone member density.

The microprocessor 38 may also be programmed so that the densitometer simultaneously performs both functions, i.e. determines both transit time and absolute attenuation of the transmitted acoustic signals, first through the member and then through the material with known acoustic properties. The densitometer may then both derive the broadband ultrasonic attenuation function and make a mathematical time comparison of the member transit time to the material transit time. The microprocessor 38 and digital signal processor 41 then relate both the time comparison along with the attenuation function to the physical properties and integrity, or density of the os calcis.

According to the invention, the microprocessor 38 is programmed so that the densitometer 10 operates in a mode whereby the need for calculating either the relative transit time or the attenuation of the acoustic signals through a material of known acoustic properties is eliminated. In order to operate in such a mode, the microprocessor 38 includes a database of normal absolute transit times which are based upon such factors as the age, height, weight, race or the sex of the individual being tested as well as the distance between the transducers or the thickness or size of the heel. This database of normal transit times can be stored in the non-volatile memory or could be stored in other media. When testing an individual in this mode, the relevant factors for the individual are placed into the microprocessor 38 to select the pertinent normal transit time based on those factors. The transducers 21 are placed on the heel bone member being tested as described above. When the actuator button 12 is pressed, the acoustic signals are transmitted through the member 32. The receive transducer 21 receives those signals after they have been transmitted through the member, and the electronics 31 then determine the heel transit time of the acoustic signals through the heel. The microprocessor 38 and digital signal processor 41 then make a mathematical comparison of the measured heel transit time to the selected database normal transit time, and relate the mathematical time comparison to the physical properties and integrity, or density of the os calcis, which is displayed.

As an alternative output of the densitometer of the present invention, the digital display 18 could also include a display corresponding to the pattern of the array of elements on the face of the transducer 21 as seen in Fig. 3. This display could then display, for each element a through 1, a gray scale image proportional to the parameter, i.e. transit time or attenuation, being measured. This image may provide a visual indication to an experienced clinician as to the physical properties of the member present in the patient.

The use of the multi-element ultrasonic transducer array for the transducers 21, as illustrated in Fig. 3, enables another advantageous feature of the instrument of Figs. 1-8. Using prior art densitometers it was often necessary to precisely position the instrument relative to the body member of the patient being measured to have useful results. The difficulty arises because of heterogenities in the bone mass and structure of actual body members. A measurement taken at one location of density may be significantly different from a measurement taken close by. Therefore prior art instruments fixed the body member precisely so that the measurement could be taken at the precise location each time.

The use of the ultrasonic transducer array obviates the need for this precise positioning. Using the instrument of Figs. 1-8, the instrument performs a pulse and response, performs the discrete Fourier transform, and generates a value for bone ultrasonic attenuation for each pair of transducer elements a through 1. Then the microprocessor 38 analyses the resulting array of bone ultrasonic density measurements to reproducibly identify the same region of interest each time. In other words, since the physical array of transducers is large enough to reliably cover at least the one common region of interest each time, the measurement is localized at the same locus each time by electrically selecting the proper location for the measurement from among the locations measured by the array. Since the instrument of Fig. 1-8 is devised for measuring the density of the os calcis as measured through the heel of a human patient, it has been found that a region of interest in the os calcis can be located reliably and repeatedly based on the comparisons of bone ultrasonic attenuation at the points in the array. The region of interest in the os calcis is identified as a local or relative minimum in bone ultrasonic attenuation and/or velocity closely adjacent the region of highest attenuation values in the body member. Thus repetitive measurements of the bone ultrasonic attenuation value at this same region of interest can be reproducibly taken even though the densitometer instrument 10 is only generally positioned at the same location for each successive measurement.

This technique of using a multiple element array to avoid position criticality is applicable to other techniques other than the determination of broadband ultrasonic attenuation as described here. The concept of using an array and comparing the array of results to determine measurement locus would be equally applicable to measurements taken of member-density based on speed of sound transit time, other measurements of attenuation or on the calculation of phase angle discussed above. The use of such a multiple-element array, with automated selection of one element in the region of interest, can also be applied to other measurement techniques useful for generating parameters related to bone member density, such as measuring speed changes in the transmitted pulse such as suggested in U.S. Patent 4,361,154 to Pratt, or measuring the frequency of a "sing-around" self-triggering pulse as suggested in U.S. Patent 3,847,141 to Hoop. The concept which permits the position independence feature is that of an array of measurements generating an array of data points from which a region of interest is selected by a reproducible criterion or several criteria. The number of elements in the array also clearly can be varied with a larger number of elements resulting in a greater accuracy in identifying the same region of interest.

In this way, the ultrasound densitometer of the present invention provides a device capable of rapid and efficient determination of the physical properties of the os calcis in vivo without the use of radiation. Because the densitometer is constructed to operate under the control of the microprocessor 38, it can be programmed to operate in one of several modes, as discussed above. This allows both for flexibility to clinical goals as well as efficient use of the device.

## Claims

1. An ultrasonic densitometer for investigating the os calcis in vivo comprising:
a transmission ultrasonic transducer array and a reception ultrasonic transducer array spaced from the transmission array so as to receive a heel (32) there between;
electrical means connected to the transmission and reception transducer arrays (21) for launching an ultrasonic pulse from the transmission array, sensing the receipt of the pulse to the reception array and measuring the transit time of the pulse passing through the heel (32) when the heel is positioned in the path of the ultrasonic pulse;
an electronic means (38) including a microprocessor operating according to a stored program to:
(i) select one of a plurality of normal transit times from an electronic database of normal transit times;
(ii) make a mathematical comparison of said member transit time of the pulse passing through the heel (32) to the selected normal transit time; and
(iii) relate said mathematical comparison to the physical properties and integrity of the os calcis of said heel.

2. An ultrasonic densitometer as claimed in claim 1, wherein the electronic database holds transit times categorized in accordance to the categories of age, height, weight, race, or sex.

3. An ultrasonic densitometer as claimed in claim 1 or claim 2, wherein the electronic database holds transit times categorized in accordance to the thickness of the heel (32).

4. An ultrasonic densitometer according to claim 1, having means (38,41,42,43) operable to determine absolute attenuation of at least one individual specific frequency component of the acoustic signal through the heel (32) when the heel (32) is positioned in the path of the pulse from its launch to its receipt, and also to determine absolute attenuation through a standard material with known acoustic properties when the standard material is positioned in the path, the microprocessor (38) being arranged to make a mathematical comparison of the absolute attenuation of an individual specific frequency component through the heel (32) to the absolute attenuation of the corresponding specific frequency component through the standard material, and to generate an output derived from the mathematical comparison relevant to the physical properties and integrity of the os calcis.

5. An ultrasonic densitometer according to claim 4, wherein the transmission ultrasonic transducer array is arranged to transmit pulses forming an acoustic signal incorporating a range of frequencies along the path;
the densitometer further includes means (38,41,42,43) operable to determine absolute attenuation of a plurality individual specific frequency components of the acoustic signal through the heel (32) when the heel (32) is positioned in the path of the signal from its launch to its receipt; and
the microprocessor (38) is arranged to make a mathematical attenuation comparison of the absolute attenuation of an individual specific component through the heel (32) to the absolute attenuation of another specific frequency component through said standard material to obtain mathematical attenuation comparisons between frequency components, and to generate an output derived from the mathematical comparison and relevant to physical properties of the os calcis.

6. A method for measuring the physical properties and integrity of the os calcis in vivo, comprising the steps of:
(a) transmitting an acoustic signal through the heel using a transmission ultrasonic transducer array and a reception ultrasonic transducer array spaced from the transmission array, the transmission array launching an ultrasonic pulse and the reception array receiving the pulse after passage through the heel;
(b) determining a heel transit time of the acoustic signal through the heel;
(c) selecting one of a plurality of normal transit times from a database of normal transit times;
(d) making a mathematical comparison of said heel transit time to the selected normal transit time; and
(e) relating said mathematical comparison to the physical properties and integrity of the os calcis.

7. A method as claimed in claim 6, wherein the database is selectable in accordance to the categories of age, height, weight, race, or sex.

8. A method as claimed in claim 6, wherein the database is selectable in accordance to the thickness of the heel and wherein the selecting of step (c) is done by identifying the width of the heel.

## Patentansprüche

1. Ultraschall-Dichtemesser zum Untersuchen des Os calcis in vivo, umfassend:
eine Ultraschall-Übertragungswandleranordnung und eine Ultraschall-Empfangswandleranordnung, die von der Übertragungsanordnung beabstandet ist, um eine Ferse (32) dazwischen aufzunehmen;
elektrische Mittel, die an die Übertragungs- und die Empfangswandleranordnung (21) angeschlossen sind, um einen Ultraschallimpuls von der Übertragungsanordnung auszusenden, den Empfang des Impulses an der Empfangsanordnung abzufühlen und die Durchgangszeit des Impulses zu messen, der durch die Ferse (32) hindurchgeht, wenn die Ferse im Weg des Ultraschallimpulses angeordnet ist;
ein elektronisches Mittel (38), das einen Mikroprozessor umfasst, der gemäß einem gespeicherten Programm arbeitet, um:
(i) eine einer Vielzahl normaler Durchgangszeiten aus einer elektronischen Datenbank normaler Durchgangszeiten auszuwählen;
(ii) einen mathematischen Vergleich der Durchgangszeit durch das Körperglied des durch die Ferse (32) hindurchgehenden Impulses mit der gewählten normalen Durchgangszeit durchzuführen; und
(iii) den mathematischen Vergleich mit den physikalischen Eigenschaften und der Integrität des Os calcis der Ferse in Beziehung zu setzen.

2. Ultraschall-Dichtemesser nach Anspruch 1, worin die elektronische Datenbank Durchgangszeiten beinhaltet, die nach den Kategorien Alter, Größe, Gewicht, Rasse oder Geschlecht kategorisiert sind.

3. Ultraschall-Dichtemesser nach Anspruch 1 oder 2, worin die elektronische Datenbank Durchgangszeiten beinhaltet, die nach der Dicke der Ferse (32) kategorisiert sind.

4. Ultraschall-Dichtemesser nach Anspruch 1, der Mittel (38, 41, 42, 43) umfasst, die betrieben werden können, um die absolute Dämpfung zumindest einer individuellen spezifischen Frequenzkomponente des akustischen Signals durch die Ferse (32) zu bestimmen, wenn die Ferse (32) im Weg des Impulses von seiner Aussendung bis zu seinem Empfang angeordnet ist, und auch, um die absolute Dämpfung durch ein Standardmaterial mit bekannten akustischen Eigenschaften zu bestimmen, wenn das Standardmaterial im Weg angeordnet ist, wobei der Mikroprozessor (38) so angeordnet ist, dass er einen mathematischen Vergleich der absoluten Dämpfung einer individuellen spezifischen Frequenzkomponente durch die Ferse (32) mit der absoluten Dämpfung der entsprechenden spezifischen Frequenzkomponente durch das Standardmaterial durchgeführt, und um eine vom mathematischen Vergleich abgeleitete Ausgangsgröße zu erzeugen, die für die physikalischen Eigenschaften und die Integrität des Os calcis relevant ist.

5. Ultraschall-Dichtemesser nach Anspruch 4, worin die Ultraschall-Übertragungswandleranordnung so angeordnet ist, dass sie Impulse überträgt, die ein akustisches Signal bilden, das einen Bereich von Frequenzen entlang des Wegs umfasst;
wobei der Dichtemesser weiters Mittel (38, 41, 42, 43) umfasst, die betreibbar sind, um die absolute Dämpfung einer Vielzahl einzelner spezifischer Frequenzkomponenten des akustischen Signals durch die Ferse (32) hindurch zu bestimmen, wenn die Ferse (32) im Weg des Signals von seiner Aussendung zu seinem Empfang angeordnet ist; und
der Mikroprozessor (38) so angeordnet ist, dass ein mathematischer Dämpfungsvergleich der absoluten Dämpfung einer einzelnen spezifischen Komponente durch die Ferse (32) mit der absoluten Dämpfung einer anderen spezifischen Frequenzkomponente durch das Standardmaterial durchgeführt wird, um mathematische Dämpfungsvergleiche zwischen Frequenzkomponenten zu erhalten und eine vom mathematischen Vergleich abgeleitete Ausgangsgröße zu erhalten, die für die physikalischen Eigenschaften des Os calcis relevant ist.

6. Verfahren zum Messen der physikalischen Eigenschaften und der Integrität des Os calcis in vivo, folgende Schritte umfassend:
(a) das Übertragen eines akustischen Signals durch die Ferse hindurch unter Einsatz einer Ultraschall-Übertragungswandleranordnung und einer Ultraschall-Empfangswandleranordnung, die von der Übertragungsanordnung beabstandet ist, wobei die Übertragungsanordnung einen Ultraschallimpuls aussendet und die Empfangsanordnung den Impuls aufnimmt nachdem er durch die Ferse hindurchgegangen ist;
(b) das Bestimmen der Fersendurchgangszeit des akustischen Signals durch die Ferse;
(c) das Auswählen einer aus einer Vielzahl anormaler Durchgangszeiten aus einer Datenbank normaler Durchgangszeiten;
(d) das Durchführen eines mathematischen Vergleichs zwischen der Fersendurchgangszeit und der gewählten normalen Durchgangszeit; und
(d) das In-Beziehung-Setzen des mathematischen Vergleichs mit den physikalischen Eigenschaften und der Integrität des Os calcis.

7. Verfahren nach Anspruch 6, worin die Datenbank nach den Kategorien Alter, Größe Gewicht, Rasse oder Geschlecht auswählbar ist.

8. Verfahren nach Anspruch 6, worin die Datenbank nach der Dicke der Ferse auswählbar ist und worin das Auswählen von Schritt (c) durch Identifizieren der Breite der Ferse erfolgt.

## Revendications

1. Densitomètre à ultrasons permettant d'étudier le calcanéum in vivo, comportant :
une matrice de transducteur d'émission d'ultrasons et une matrice de transducteur de réception d'ultrasons disposée à distance de la matrice d'émission de manière à permettre de placer un talon (32) entre elles;
des moyens électriques reliés aux matrices (21) de transducteur d'émission et de réception pour faire délivrer une impulsion d'ultrasons par la matrice d'émission, détecter la réception de l'impulsion sur la matrice de réception et mesurer le temps de transit de l'impulsion traversant le talon (32) lorsque le talon est disposé dans le parcours de l'impulsion d'ultrasons;
un moyen électronique (38) comprenant un microprocesseur travaillant suivant un programme enregistré, pour :
(i) sélectionner dans une base de données électronique de durées normales de transit l'une parmi plusieurs durées normales de transit;
(ii) effectuer une comparaison mathématique entre ladite durée de transit de l'impulsion traversant le talon (32) et la durée normale du transit sélectionnée; et
(iii) associer ladite comparaison mathématique aux propriétés physiques et à l'intégrité du calcanéum dudit talon.

2. Densitomètre à ultrasons selon la revendication 1, dans lequel la base de données électronique contient des durées de transit classées en catégories en fonction de catégories d'âge, de taille, de poids, de race ou de sexe.

3. Densitomètre à ultrasons selon la revendication 1 ou la revendication 2, dans lequel la base de données électronique contient des durées de transit classées en catégories en fonction de l'épaisseur du talon (32).

4. Densitomètre à ultrasons selon la revendication 1, présentant des moyens (38, 41, 42, 43) aptes à travailler pour déterminer l'atténuation absolue d'au moins une composante individuelle, de fréquence spécifique, du signal acoustique à travers le talon (32) lorsque le talon (32) est placé dans le parcours de l'impulsion entre son émission et sa réception, et également pour déterminer une atténuation absolue par un matériau standard à propriétés acoustiques connues lorsque le matériau standard est placé dans le parcours, le microprocesseur (38) étant agencé pour effectuer une comparaison mathématique entre l'atténuation absolue d'une composante individuelle de fréquence spécifique à travers le talon (32) et l'atténuation absolue de la composante correspondante de fréquence spécifique à travers le matériau standard, et pour générer une sortie dérivée de la comparaison mathématique et associée aux propriétés physiques et à l'intégrité du calcanéum.

5. Densitomètre à ultrasons selon la revendication 4, dans lequel la matrice de transducteur d'émission d'ultrasons est agencée pour émettre des impulsions sur le parcours, lesquelles impulsions forment un signal acoustique incorporant une plage de fréquences;
le densitomètre comprenant en outre des moyens (38, 41, 42, 43) aptes à travailler pour déterminer l'atténuation absolue d'une pluralité de composantes individuelles de fréquence spécifique du signal acoustique à travers le talon (32) lorsque le talon (32) est placé dans le parcours du signal entre son émission et sa réception; et
le microprocesseur (38) étant agencé pour effectuer une comparaison mathématique de l'atténuation entre l'atténuation absolue d'une composante individuelle spécifique à travers le talon (32) et l'atténuation absolue d'une autre composante de fréquence spécifique à travers ledit matériau standard, pour obtenir des comparaisons mathématiques entre les atténuations des composantes de fréquence et pour générer une sortie dérivée de la comparaison mathématique et associée à des propriétés physiques du calcanéum.

6. Procédé de mesure in vivo des propriétés physiques et de l'intégrité du calcanéum, lequel procédé comporte les étapes consistant à :
(a) émettre un signal acoustique à travers le talon en utilisant une matrice de transducteur d'émission d'ultrasons et une matrice de transducteur de réception d'ultrasons située à distance de la matrice d'émission, la matrice d'émission émettant une impulsion d'ultrasons et la matrice de réception recevant l'impulsion après son passage à travers le talon;
(b) déterminer une durée du transit dans le talon du signal acoustique ayant traversé le talon;
(c) sélectionner l'une parmi plusieurs durées normales de transit dans une base de données de durées normales de transit;
(d) effectuer une comparaison mathématique entre ladite durée de transit dans le talon et la durée normale de transit sélectionnée; et
(e) associer ladite comparaison mathématique aux propriétés physiques et à l'intégrité du calcanéum.

7. Procédé selon la revendication 6, dans lequel la base de données peut être sélectionnée en accord aux catégories d'âge, de taille, de poids, de race ou de sexe.

8. Procédé selon la revendication 6, dans lequel la base de données peut être sélectionnée en accord à l'épaisseur du talon, et dans lequel la sélection de l'étape (c) est effectuée par identification de la largeur du talon.
